# FASCICULE DE BREVET EUROPEEN

(11) **EP 2 544 613 B1**
(45) Date de publication et mention de la délivrance du brevet: **23.08.2017**
(21) Numéro de dépôt: 11714351.1
(22) Date de dépôt: 08.03.2011
(51) Int. Cl.: A61B 17/80

(54) **PLAQUE D'OSTEOSYNTHESE ARTICULEE**
OSTEOSYNTHESEPLATTE MIT SCHARNIER
HINGED OSTEOSYNTHESIS PLATE

(30) Priorité: 08.03.2010 FR 1000937
(43) Date de publication de la demande: 16.01.2013
(73) Titulaire: Stryker European Holdings I, LLC, Kalamazoo, MI 49002 (US)
(72) Inventeur: PRANDI, Bernard, F-35700 Rennes (FR); CHICK, Grégoire, CH-1227 Carouge-Genève (CH); COGNET, Jean-Michel, F-51100 Reims (FR); MARTINACHE, Xavier, F-51100 Reims (FR); PAPALOÏZOS, Michaël, CH-1204 Genève (CH); TCHURUKDICHIAN, Alain, F-21000 Dijon (FR)
(74) Mandataire: Regimbeau
(86) Numéro de dépôt international: PCT/FR2011/000124
(87) Numéro de publication internationale: WO 2011/110757

(56) Documents cités:
- WO-A2-2004/045389
- JP-A- 9 206 310
- US-A- 6 129 728
- US-A1- 2006 089 648
- US-A1- 2007 299 448
- US-A1- 2009 082 813

## Description

La présente invention concerne une plaque d'ostéosynthèse pour réduction d'une fracture distale d'un os du type radius, la plaque comportant une partie épiphysaire élargie en forme de palette munie de trous de fixation pour vis d'ancrage et une partie diaphysaire allongée également munie de trous pour vis d'ancrage.

Par partie épiphysaire on entend la partie située du côté distal de l'os ou épiphyse fracturée, et par partie diaphysaire, la partie située du côté proximal ou diaphyse du corps de l'os concerné.

L'invention trouve une application particulièrement importante bien que non exclusive dans le domaine de la réduction et/ou de la fixation provisoire d'une partie fracturée d'un radius sur le reste de l'os, la plaque pouvant éventuellement être retirée après consolidation de la fracture. Une telle plaque va également permettre la rééducation plus rapide de l'articulation en la sollicitant, pour éviter des phénomènes ultérieurs tels que l'arthrose.

On connaît déjà, dans le cas de fractures distales du radius, des plaques permettant un positionnement en longueur grâce à un trou oblong. De telles plaques, qui ramènent le fragment distal à la bonne distance et le fixent en réglant en longueur la plaque avant de la verrouiller dans la diaphyse, n'autorisent cependant aucun ajustement latéral.

Or, il se trouve que dans certains cas, il est nécessaire de fixer d'abord les fragments distaux du radius sur la plaque. Ce n'est qu'ensuite que la fracture va être réduite sur la diaphyse.

On ne peut cependant jamais être certain de l'orientation de la plaque, qui n'est dès lors pas toujours dans l'axe du radius.

Il peut alors y avoir un dépassement côté proximal de la partie diaphysaire allongée et donc gêne pour le patient. Le chirurgien ne peut pas corriger ce léger défaut de positionnement latéral sans repositionner la partie épiphysaire ce qui va nécessiter de repercer à côté des premiers trous pour se décaler. En pratique ce n'est pas possible car les trous sont alors trop proches.

On connaît également (WO 2004/045389 ou US 2009/0275947) des plaques munies de moyens pivotant permettant d'ajuster en rotation la partie diaphysaire par rapport à la partie épiphysaire.

Mais de tels moyens sont soit trop compliqués, soit peu solides et/ou trop encombrants en hauteur.

L'objet de la présente invention est de proposer une plaque d'ostéosynthèse répondant mieux que celles antérieurement connues aux exigences de la pratique, notamment en ce qu'elle permet de réorienter la partie diaphysaire allongée malgré la fixation initiale de la partie épiphysaire lors de l'opération, tout en présentant un encombrement faible en épaisseur, et une excellente fixation de la partie épiphysaire sur les deux parties d'os à réduire.

Pour ce faire, le mode de réalisation de l'invention plus particulièrement décrit, non seulement se propose d'orienter entre elles, de façon modifiable dans le plan de la partie diaphysaire allongée, les deux parties à savoir la partie épiphysaire et la partie diaphysaire, mais ce en proposant une fixation simple et solide de la partie épiphysaire.

Une telle possibilité d'orientation permet un réglage du positionnement de la partie diaphysaire de la plaque, tout en autorisant un déplacement longitudinal par le biais d'un trou oblong, puis un verrouillage dans la bonne position, alors que les deux parties d'os sont réduites et fixées solidement l'une à l'autre.

L'invention est bien entendu utilisable sur tout type de fracture d'extrémité d'os allongé ou long, ou d'ostéosynthèse, et non pas seulement sur une extrémité de radius.

Dans ce but la présente invention propose essentiellement une plaque d'ostéosynthèse telle que décrite dans les revendications annexées.

La partie diaphysaire de la plaque pouvant s'orienter pour être fixée de façon réglable par rapport à la partie distale en forme de palette, cela permet d'éviter un dépassement latéral par rapport au corps de l'os, dépassement qui aurait occasionné une gêne pour le patient.

La partie épiphysaire est agencée pour être rigidement fixée sur les deux parties d'os.

Les portions de fixation sont ainsi agencées pour être en contact par leurs faces inférieures directement avec l'os à réduire.

De la sorte, en étant fixée par le biais des vis d'ancrage mettant la partie épiphysaire directement en pression entre la tête des vis et l'os, une fixation d'une grande solidité entre les deux parties d'os est obtenue.

Dans des modes de réalisation avantageux, on a de plus recours à l'une et/ou à l'autre des dispositions suivantes :
- la plaque comporte des moyens de verrouillage dans la position angulaire choisie, rendant l'articulation blocable dans cette position.

Par articulation blocable, on entend une articulation permettant un réglage en orientation, qui une fois ladite orientation choisie, permet ensuite de bloquer rigidement les deux parties l'une par rapport à l'autre ;
- les moyens de verrouillage de la partie épiphysaire avec la partie diaphysaire comprennent des moyens de blocage surfaciques espacés longitudinalement avec un système de blocage réglable par vis de blocage. Par moyens surfaciques il faut entendre des moyens de jonction et/ou de coopération de l'un par rapport à l'autre par l'intermédiaire d'une portion de contact régulière ou non s'étendant sur une surface par exemple de 0,5 cm² ou plus ;
- pour former le système de blocage réglable l'une des extrémités de jonction entre parties diaphysaire et épiphysaire de la plaque comporte un téton en vis à vis d'une lumière située dans l'autre extrémité de jonction complémentaire entre partie épiphysaire et diaphysaire, avec laquelle il coopère à glissement, et une vis de fixation du téton sur l'autre extrémité au travers de ladite lumière ;
- la partie diaphysaire est sensiblement allongée dans un premier plan et la partie épiphysaire est située principalement dans un deuxième plan incliné par rapport au plan de ladite partie diaphysaire.

L'angle donné entre les plans des parties diaphysaire et épiphysaire permet de mieux s'adapter à la configuration de l'os. Il est par exemple de quelques degrés par exemple 5 à 15°, et avantageusement entre 8 et 10°.

Le fait d'être principalement situé dans un plan incliné permet notamment à la jonction entre plaques d'être droite ;
- les deux valeurs d'angles déterminées dans le plan diaphysaire sont +15° et -15° par rapport à la position d'alignement central, autorisant toutes les positions angulaires entre les deux valeurs de façon continue ou quasi-continue. Les valeurs extrêmes peuvent être également inférieures, par exemple +10° et -10° ;
- les parties épiphysaires et diaphysaires sont amovibles l'une par rapport à l'autre. En d'autres termes elles ne sont pas structurellement solidaires, mais séparées l'une de l'autre avant montage et fixation de l'une sur l'autre ;
- pour former les moyens surfaciques de blocage, chacune des parties épiphysaire et diaphysaire comprend une extrémité de jonction avec l'autre partie présentant une surface de coopération de l'une avec l'autre, et des moyens de blocage en rotation desdites extrémités l'une par rapport à l'autre ;

- les extrémités de jonction en vis à vis sont amincies et superposées pour ne pas présenter de surépaisseur par rapport au reste de la partie diaphysaire une fois jointes ;
- les moyens surfaciques de blocage comprennent deux surfaces crantées en vis à vis chacune respectivement solidaires d'une extrémité de jonction, propres à tourner l'une par rapport à l'autre à distance et à se bloquer l'une en contact de l'autre et une vis de blocage de l'une sur l'autre.

En d'autres termes les extrémités de jonction comprennent des surfaces crantées identiques en regard l'une de l'autre, une mobilité en rotation existant quand elles sont écartées l'une de l'autre, un blocage étant par contre obtenu quand elles sont rapprochées et en contact l'une avec l'autre, de sorte que les crans des deux surfaces en vis à vis se pénètrent les uns les autres.

Le blocage se fait par l'intermédiaire d'une vis de façon connue ;
- l'une des extrémités de jonction comporte un téton en vis à vis d'une lumière située dans l'autre extrémité de jonction avec laquelle il coopère à glissement, et une vis de fixation du téton sur l'autre extrémité au travers de ladite lumière.

Les vis utilisées sont agencées pour se verrouiller en fin de course pour éviter toute migration de façon connue en elle-même.

Par l'une des extrémités de jonction porteuses du téton, on entend dans le mode plus particulièrement décrit une extrémité de la partie épiphysaire, et par l'autre extrémité de jonction munie de la lumière une extrémité de la partie diaphysaire.

Mais cela peut être l'inverse, la lumière étant sur la partie diaphysaire et le téton sur la partie épiphysaire.

La présente invention sera mieux comprise à la lecture de la description qui suit d'un mode de réalisation donné ci-après à titre d'exemple non limitatif.

L'invention se réfère aux figures qui l'accompagnent dans lesquelles :
Les figures 1A, 1B et 1C montrent un mode de réalisation de plaque articulée selon l'invention dans trois positions angulaires respectives, respectivement à 0 degré, à 5 degrés et à 10 degrés par rapport à une position d'alignement central.
Les figures 2A et 2B montrent en perspective axionométrique, respectivement par le dessus et par le dessous, les extrémités de jonction entre les parties faisant apparaître les moyens de blocage en rotation selon le mode de réalisation de l'invention plus particulièrement décrit ici.
Les figures 3A à 3D donnent les étapes successives de mise en place d'une plaque d'ostéosynthèse selon le mode de réalisation de la figure 1.

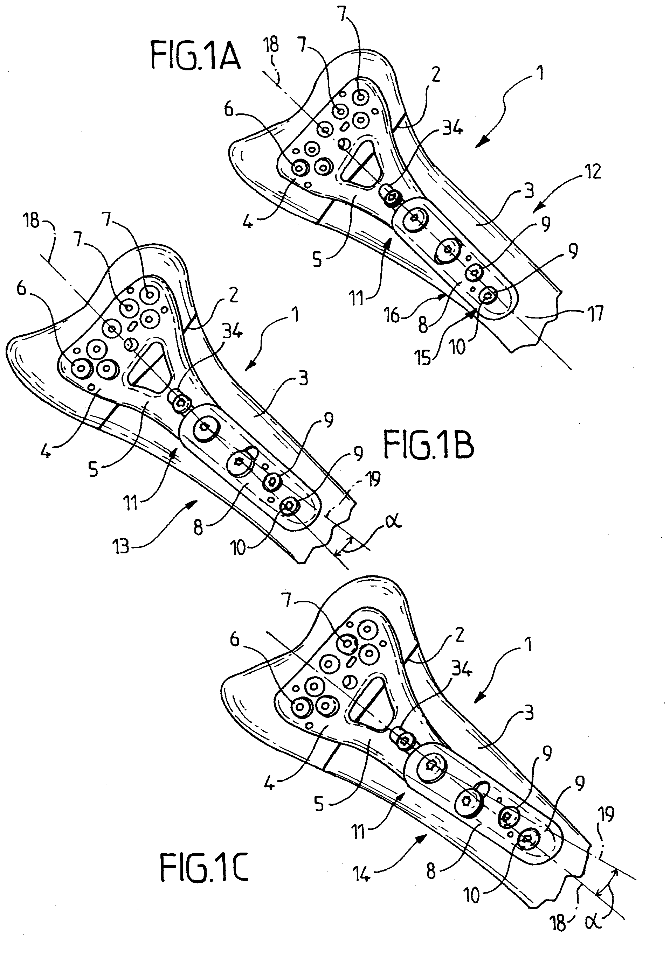

Les figures 1 montrent une plaque d'ostéosynthèse 1 pour réduction d'une fracture distale 2 du radius 3.

La plaque 1 comporte une partie épiphysaire élargie 4 en forme de palette sensiblement trapézoïdale par exemple munie d'une zone centrale évidée 5 à la base de la partie épiphysaire.

La partie épiphysaire 4 est munie de trous de fixation 6 par des premières vis d'ancrage 7, de façon connue en elle-même.

Elle comporte également une partie diaphysaire 8 allongée, par exemple sensiblement rectangulaire, munie de seconds trous 9 de fixation par secondes vis d'ancrage 10.

Selon le mode de réalisation plus particulièrement décrit ici, la plaque 1 comporte de plus un système 11 d'articulation entre les deux parties d'amplitude ± 15°.

Plus précisément les figures 1A à 1C montrent les parties épiphysaire et diaphysaire lorsqu'elles sont en position d'alignement central (position 12 - figure 1A), présentant un angle de 5° (position 13 - figure 1B) ou de 10° (position 14 - figure 1C)).

On voit que dans le cas de l'alignement central (figure 1A), le bord externe 15 de l'extrémité de la partie diaphysaire 8 est très proche du bord 16 de la partie proximale 17 de l'os ce qui peut occasionner une gêne. Dans la position 13 (figure 1B), l'angle α entre l'axe ou ligne 18 d'alignement central, avec l'axe 19 de la partie diaphysaire est de 5°, et de 10° dans la position 14 (figure 1C).

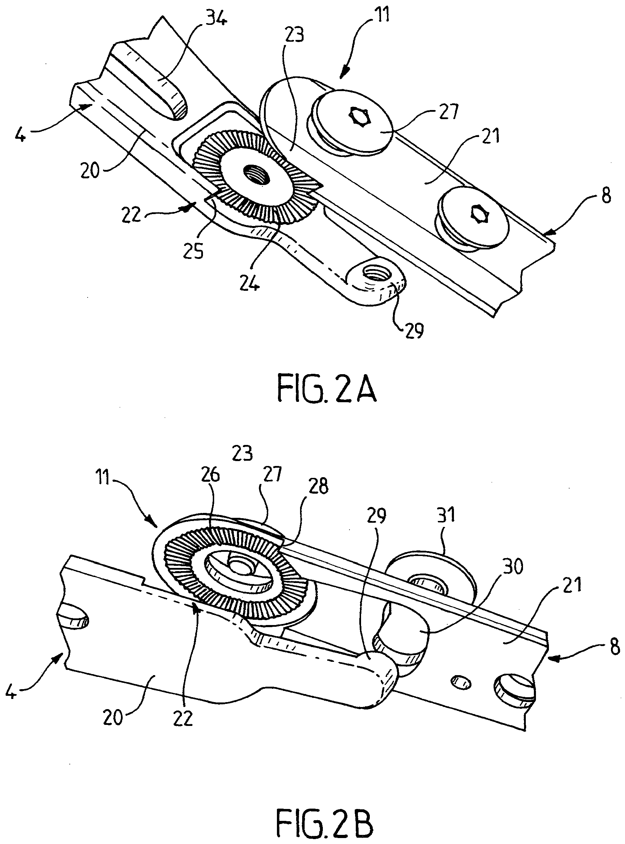

Les figures 2A et 2B montrent plus précisément le système d'articulation 11 de la figure 1, entre les extrémités 20 et 21, respectivement des parties épiphysaire 4 et diaphysaire 8. Chaque extrémité 20 et 21 est affinée dans sa partie distale respective 22, 23 pour se raccorder sans surépaisseur.

Plus précisément l'extrémité 20 comporte une surface de coopération de jonction 24, crantée, circulaire, comprenant de part et d'autre des butées 25 de l'extrémité en rotation.

L'autre extrémité 21 en vis à vis comprend également une surface de jonction 26 circulaire crantée en regard, agencée pour tourner par rapport à la surface crantée 24 en vis à vis autour d'un axe central.

Une vis 27 vient plaquer les deux parties d'extrémité 20 et 21 l'une sur l'autre et verrouiller le système dans la position angulaire choisie.

L'extrémité 21 comporte également deux butées 28 venant en butée avec les butées 25 de l'extrémité de la partie épiphysaire, lorsque les angles extrêmes de +15° par exemple ou -15° sont atteints.

L'extrémité 20 comporte par ailleurs un téton 29 de la partie inférieure, qui est ici dans ce mode de réalisation, la partie épiphysaire 4 agencée pour venir s'encastrer dans une lumière 30 en arc de cercle appartenant à la partie supérieure 21.

Une vis de verrouillage 31 vient compléter ces moyens de fixation d'une extrémité sur l'autre, en se vissant dans le téton en vis à vis lors de la fixation au travers de la lumière 30, de la tête de
la vis une collerette vient se bloquer sur le dessus de la partie 21 lorsque les deux extrémités sont accolées l'une à l'autre et verrouillées en rotation.

Les vis 27 et 31 sont agencées pour se verrouiller en fin de course pour éviter toute migration, de façon connue en elle-même.

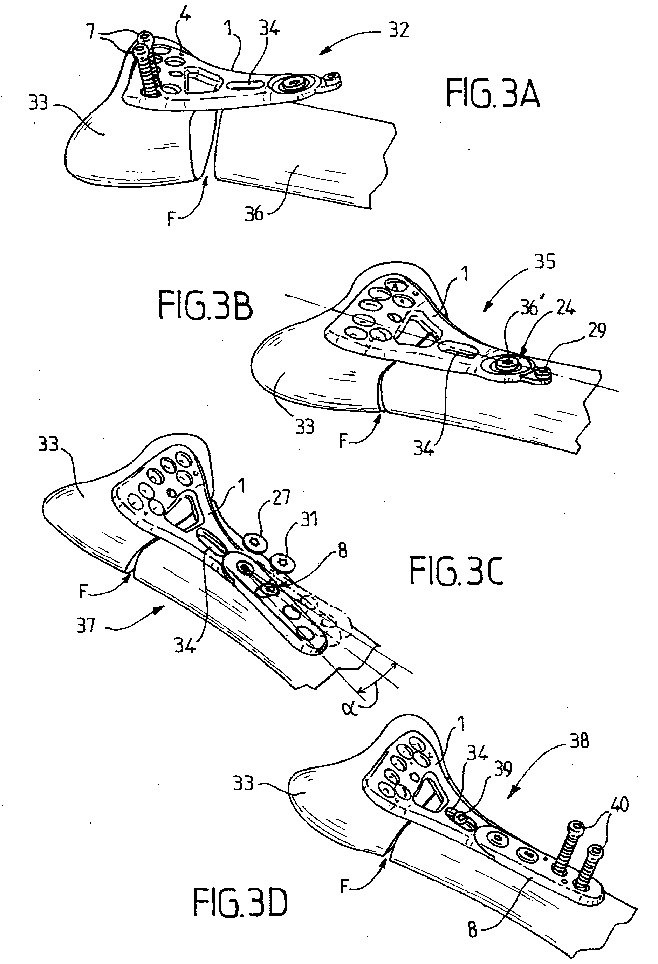

On va maintenant décrire en référence à la figure 3 la mise en place d'une plaque selon le mode de réalisation plus particulièrement décrit ici.

Dans la suite on utilisera les mêmes numéros de référence pour désigner les mêmes éléments que dans les figures décrites ci-avant.

Tout d'abord (étape 32 - figure 3A) on met en place à l'oeil, la plaque 1 sur la partie épiphysaire 33 de l'os, ce qui est particulièrement utile en cas de fracture communitive F.

Pour se faire on fixe la partie épiphysaire 4 par des vis d'ancrage 7, en réglant au mieux.

Comme représenté sur la figure 3A la partie épiphysaire 4 comporte également un trou oblong 34, qui permet le réglage longitudinal de façon connue en elle-même.

Puis (étape 35 figure 3B) on réduit la fracture F à l'aide de la plaque, en rapprochant la partie fracturée 33 du reste de l'os 36. Mais à ce stade on ne contrôle pas vraiment l'axe longitudinal 18 de la plaque posée qui passe par le centre 36' de la surface circulaire crantée de jonction 24.

En effet seule la partie épiphysaire, comme indiqué sur la figure 3B est placée pour l'instant sur l'os.

Puis on vient apposer (étape 37 - figure 3C) la partie diaphysaire 8 en réglant l'angle α souhaité, pour corriger l'axe de cette partie de la plaque par rapport à l'os.

Une fois correctement placée on verrouille, par l'intermédiaire des deux vis de verrouillage 27 et 31 comme décrit ci-avant.

Enfin on affine la position longitudinale (étape 38 - figure 3D) grâce au trou oblong 34 et à la vis 39 avant de fixer définitivement la partie diaphysaire 8 par les vis 40.

De la sorte, la partie épiphysaire est directement fixée, d'une part à la partie fracturée d'os 33 et d'autre part au reste de l'os 36, indépendamment de la partie diaphysaire orientable qui termine la fixation en longueur sur l'os 36, de façon autorigidifiée par le système de double vis 27 et 31.

A titre d'exemple non limitatif la longueur de l'axe de rotation de la vis 27 est de 5 à 10 mm, la profondeur des crans de l'ordre de 0,2 mm, le nombre de crans étant compris entre 50 et 100, pour une longueur de partie amincie de 10 à 15 mm.

Comme il va de soi et comme il résulte également de ce qui précède, la présente invention n'est pas limitée au mode de réalisation plus particulièrement décrit. Elle en embrasse au contraire toutes les variantes et notamment celles où les angles ne sont pas de ±15° mais ±10° et/ou ±20°, celle où la partie épiphysaire et la partie diaphysaire sont différentes de celles décrites et/ou sont en matériaux différents, celles où il existe deux tétons en vis à vis de deux lumières respectivement situées dans l'autre extrémité avec lesquels ils coopèrent à glissement, à savoir un téton sur la partie éphiphysaire et un téton sur la partie diaphysaire, et une lumière sur les parties en vis à vis, les tétons (et donc les lumières) étant située de part et d'autre, par exemple symétriquement de l'axe de rotation de l'articulation et se verrouillant avec des vis de fixation du téton au travers de la lumière correspondante.

## Revendications

1. Système pour réduction d'une fracture distale (2) d'os (3) allongé, entre une partie fracturée distale (33) de l'os et une partie proximale (36), comprenant :
une plaque (1) d'ostéosynthèse comportant une partie épiphysaire (4) élargie en forme de palette munie de premiers trous (6) de fixation pour premières vis d'ancrage (7) et une partie diaphysaire (8) allongée munie de seconds trous (9) de fixation pour secondes vis d'ancrage (10), ladite plaque comportant une articulation entre la partie épiphysaire et la partie diaphysaire dans le plan de ladite partie diaphysaire entre deux valeurs d'angle déterminées par rapport à une position d'alignement central (18); et
une troisième vis d'ancrage (39) ;
la partie épiphysaire (4) comprenant d'un côté une première portion de fixation formant une extrémité élargie distale munie desdits premiers trous (6) de fixation sur la partie distale (33) de l'os fracturé, et de l'autre côté une deuxième portion de fixation formant une extrémité rétrécie proximale,
**caractérisé en ce que** ladite extrémité rétrécie proximale est munie d'un premier trou oblong (34) de fixation sur la partie proximale (36) de l'os destiné à recevoir la troisième vis d'ancrage (39), pour fixer la partie épiphysaire (4) à chacune des parties fracturées distale (33) et proximale (36) de l'os (3) fracturé de sorte que la partie épiphysaire est inamovible vis-à-vis de l'os, le premier trou oblong (34) étant tel que les parties distale (33) et proximale (36) de l'os fracturé sont mobiles l'une par rapport à l'autre avant l'insertion de la troisième vis d'ancrage (39) dans la partie distale (33), et immobiles l'une par rapport à l'autre après l'insertion de la troisième vis d'ancrage (39) dans la partie distale (33),
et **en ce que** l'extrémité rétrécie de la partie épiphysaire comprend en outre une vis de blocage (27) et une vis de fixation (31), la vis de blocage (27) définissant un axe de rotation entre les parties épiphysaire et diaphysaire et étant adaptée pour verrouiller la partie épiphysaire (4) avec la partie diaphysaire (8), la vis de fixation (31) étant agencée pour joindre la partie épiphysaire (4) à la partie diaphysaire (8) à travers un deuxième trou oblong (30) formé dans la partie diaphysaire (8).

2. Système selon la revendication 1, **caractérisé en ce qu'**il comporte des moyens (24, 26, 27) de verrouillage de la partie épiphysaire (4) avec la partie diaphysaire (8) dans une position angulaire choisie.

3. Système selon la revendication 2, **caractérisé en ce que** les moyens de verrouillage de la partie épiphysaire (4) avec la partie diaphysaire (8) dans la position angulaire choisie comprennent des moyens surfaciques (24, 26) de blocage espacés longitudinalement avec un système (11) de blocage réglable par la vis de blocage (27).

4. Système selon la revendication 3, **caractérisé en ce que** pour former le système (11) de blocage réglable l'extrémité de la partie épiphysaire (4) de jonction avec la partie diaphysaire (8) comporte un téton (29) en vis à vis du deuxième trou oblong (30) situé dans une extrémité (21) de la partie diaphysaire (8) de jonction de la partie diaphysaire (8) avec la partie épiphysaire (4) avec laquelle il coopère à glissement, et **en ce que** la vis de fixation (31) fixe le téton sur l'extrémité (21) de la partie diaphysaire (8) au travers du deuxième trou oblong (30).

5. Système selon la revendication 3, **caractérisé en ce que**, pour former les moyens surfaciques de blocage, chacune des parties épiphysaire et diaphysaire comprend une extrémité (20, 21) de jonction avec l'autre partie présentant une surface de coopération (24, 26) de l'une avec l'autre, et des moyens (24, 26 ; 27, 31) de blocage en rotation desdites extrémités l'une par rapport à l'autre pour former lesdits moyens de verrouillage.

6. Système selon la revendication 5, **caractérisé en ce que** les extrémités (20, 21) de jonction en vis à vis sont amincies et superposées pour ne pas présenter de surépaisseur par rapport au reste de la partie diaphysaire une fois jointes.

7. Système selon l'une des revendications 5 ou 6, **caractérisé en ce que** les moyens de blocage comprennent deux surfaces crantées (24, 26) en vis à vis chacune respectivement solidaires d'une extrémité, propres à tourner l'une par rapport à l'autre d'une distance et à se bloquer l'une en contact de l'autre, et une vis mettre en prise l'une des surfaces crantées avec l'autre.

8. Système selon l'une quelconque des revendications précédentes, **caractérisé en ce que** la partie diaphysaire (8) est sensiblement allongée dans un premier plan et la partie épiphysaire (4) est située principalement dans un deuxième plan incliné par rapport au plan de ladite partie diaphysaire.

9. Système selon l'une quelconque des revendications précédentes, **caractérisé en ce que** les deux valeurs d'angles déterminées dans le plan diaphysaire sont +15° et - 15°, par rapport à la position d'alignement central.

10. Système selon l'une quelconque des revendications précédentes, **caractérisé en ce que** les parties épiphysaire (4) et diaphysaire (8) sont amovibles l'une par rapport à l'autre.

11. Système selon l'une quelconque des revendications précédentes, **caractérisé en ce que** la partie diaphysaire (8) comprend d'un côté une extrémité externe (21) adjacente à la deuxième portion de fixation de la partie épiphysaire, l'extrémité externe (21) ayant le deuxième trou oblong (30) positionné devant l'extrémité rétrécie proximale (22) de la partie épiphysaire, le deuxième trou oblong ayant une dimension transversale s'étendant par rapport à l'axe longitudinal, l'extrémité rétrécie proximale de la partie épiphysaire s'étendant sous l'extrémité externe de la partie diaphysaire et incluant un siège de téton (29), sur une extrémité de cette dernière, ledit téton (29) ayant un axe situé dans ledit siège et une tête s'étendant au-dessus en contact avec l'extrémité externe de la partie diaphysaire et **en ce que** le deuxième trou oblong (30) est dimensionné pour autoriser l'engagement de téton avec la partie épiphysaire au travers du deuxième trou oblong durant le pivotement relatif des parties épiphysaire et diaphysaire.

12. Système selon la revendication 1, dans lequel la plaque d'ostéosynthèse comprend en outre une fenêtre entre les premiers trous (6) de fixation et le premier trou oblong (34), la fenêtre étant configurée pour être positionnée au-dessus de la fracture distale (2) de sorte que la fracture distale est visible à travers la fenêtre lors d'un mouvement de la partie distale (33) et de la partie proximale (36) l'une par rapport à l'autre.

## Patentansprüche

1. System zur Reduzierung einer distalen Fraktur (2) eines langen Knochens (3) zwischen einem gebrochenen distalen Teil des Knochens (33) und einem proximalen Teil (36), umfassend:
eine Osteosyntheseplatte (1), aufweisend einen palettenförmig erweiterten epiphysären Teil (4), ausgestattet mit ersten Befestigungslöchern (6) für erste Verankerungsschrauben (7), und einen länglichen diaphysären Teil (8), ausgestattet mit zweiten Befestigungslöchern (9) für zweite Verankerungsschrauben (10), wobei die Platte ein Gelenk zwischen dem epiphysären Teil und dem diaphysären Teil in der Ebene des diaphysären Teils zwischen zwei Winkelwerten, bestimmt in Bezug auf eine mittige Ausrichtungsposition (18), aufweist, und
eine dritte Verankerungsschraube (39),
wobei der epiphysäre Teil (4) auf der einen Seite einen ersten Befestigungsabschnitt umfasst, der ein erweitertes distales Ende bildet, ausgestattet mit ersten Befestigungslöchern (6) auf dem distalen Teil (33) des gebrochenen Knochens, und auf der anderen Seite einen zweiten Befestigungsteil, der ein verengtes proximales Ende bildet,
**dadurch gekennzeichnet, dass** das verengte proximale Ende mit einem ersten länglichen Befestigungsloch (34) auf dem proximalen Teil (36) des Knochens ausgestattet ist, das bestimmt ist, die dritte Verankerungsschraube (39) zu empfangen, um den epiphysären Teil (4) am gebrochenen distalen (33) und proximalen Teil (36) des gebrochenen Knochens (3) derart zu befestigen, dass der epiphysäre Teil gegenüber dem Knochen unlösbar ist, wobei das erste längliche Loch (34) derart ist, dass der distale (33) und proximale Teil (36) des gebrochenen Knochens vor dem Einsetzen der dritten Verankerungsschraube (39) in den distalen Teil (33) in Bezug zueinander bewegbar und nach dem Einsetzen der dritten Verankerungsschraube (39) in den distalen Teil (33) in Bezug zueinander unbewegbar sind,
und dass das verengte Ende des epiphysären Teils ferner eine Blockierschraube (27) und eine Befestigungsschraube (31) umfasst, wobei die Blockierschraube (27) eine Rotationsachse zwischen dem epiphysären und diaphysären Teil definiert und ausgebildet ist, um den epiphysären Teil (4) mit dem diaphysären Teil (8) zu verriegeln, wobei die Befestigungsschraube (31) ausgebildet ist, um den epiphysären Teil (4) mit dem diaphysären Teil (8) durch ein zweites längliches Loch (30), gebildet im diaphysären Teil (8), zu verbinden.

2. System nach Anspruch 1, **dadurch gekennzeichnet, dass** es Verriegelungsmittel (24, 26, 27) des epiphysären Teils (4) mit dem diaphysären Teil (8) in einer gewählten Winkelposition aufweist.

3. System nach Anspruch 2, **dadurch gekennzeichnet, dass** die Verriegelungsmittel des epiphysären Teils (4) mit dem diaphysären Teil (8) in der gewählten Winkelposition flächige Blockiermittel (24, 26) umfassen, die mit einem von der Blockierschraube (27) einstellbaren Blockiersystem (11) längs beabstandet sind.

4. System nach Anspruch 3, **dadurch gekennzeichnet, dass**, um das einstellbare Blockiersystem (11) zu bilden, das Verbindungsende des epiphysären Teils (4) mit dem diaphysären Teil (8) einen Ansatz (29) gegenüber dem zweiten länglichen Lochs (30), befindlich in einem Verbindungsende (21) des diaphysären Teils (8) des diaphysären Teils (8) mit dem epiphysären Teil (4), mit welchem er gleitend zusammenwirkt, aufweist, und dass die Befestigungsschraube (31) den Ansatz auf dem Ende (21) des diaphysären Teils (8) durch das zweite längliche Loch (30) befestigt.

5. System nach Anspruch 3, **dadurch gekennzeichnet, dass**, um die flächigen Blockiermittel zu bilden, sowohl der epiphysäre als auch der diaphysäre Teil ein Verbindungsende (20, 21) mit dem anderen Teil umfassen, aufweisend eine Kooperationsfläche (24, 26) der einen mit der anderen, und Rotationsblockiermittel (24, 26; 27, 31) der Enden in Bezug zueinander, um die Verriegelungsmittel zu bilden.

6. System nach Anspruch 5, **dadurch gekennzeichnet, dass** die gegenüberliegenden Verbindungsenden (20, 21) verschlankt sind und übereinanderliegen, um nach der Verbindung in Bezug zum Rest des diaphysären Teils keine Überdicke aufzuweisen.

7. System nach einem der Ansprüche 5 oder 6, **dadurch gekennzeichnet, dass** die Blockiermittel zwei gegenüberliegende Rastflächen (24, 26) umfassen, von denen jede mit jeweils einem Ende verbunden ist, die imstande sind, zueinander in einer Distanz zu drehen und im Kontakt mit der anderen zu blockieren, und eine Schraube, welche in eine der Rastflächen mit der anderen eingreift.

8. System nach einem der vorangehenden Ansprüche, **dadurch gekennzeichnet, dass** der diaphysäre Teil (8) etwa länglich ist in einer ersten Ebene und sich der epiphysäre Teil (4) hauptsächlich in einer zweiten Ebene befindet, die in Bezug zur Ebene des diaphysären Teils geneigt ist.

9. System nach einem der vorangehenden Ansprüche, **dadurch gekennzeichnet, dass** die zwei bestimmten Winkelwerte in der diaphysären Ebene in Bezug zur mittigen Ausrichtungsposition +15° und -15° betragen.

10. System nach einem der vorangehenden Ansprüche, **dadurch gekennzeichnet, dass** der epiphysäre (4) und diaphysäre (8) Teil in Bezug zueinander lösbar sind.

11. System nach einem der vorangehenden Ansprüche, **dadurch gekennzeichnet, dass** der diaphysäre Teil (8) auf der einen Seite ein äußeres Ende (21) neben dem zweiten Befestigungsabschnitt des epiphysären Teils umfasst, wobei das äußere Ende (21) das zweite längliche Loch (30) hat, das vor dem verengten proximalen Ende (22) des epiphysären Teils positioniert ist, wobei das zweite längliche Loch eine transversale Abmessung hat, die sich in Bezug zur Längsachse erstreckt, wobei sich das verengte proximale Ende des epiphysären Teils unter dem äußeren Ende des diaphysären Teils erstreckt und einen Sitz eines Ansatzes (29) auf einem Ende dieses Letztgenannten einschließt, wobei der Ansatz (29) eine Achse hat, die sich in dem Sitz befindet, und einen Kopf, der sich darüber im Kontakt mit dem äußeren Ende des diaphysären Teils erstreckt, und dass das zweite längliche Loch (30) bemessen ist, um das Engreifen des Ansatzes in den epiphysären Teil durch das zweite längliche Loch während des relativen Schwenkens des epiphysären und diaphysären Teils zu erlauben.

12. System nach Anspruch 1, wobei die Osteosyntheseplatte ferner ein Fenster zwischen dem ersten Befestigungsloch (6) und dem ersten länglichen Loch (34) umfasst, wobei das Fenster konfiguriert ist, um über der distalen Fraktur (2) derart positioniert zu sein, dass die distale Fraktur bei einer Bewegung des distalen Teils (33) und des proximalen Teils (36) zueinander durch das Fenster zu sehen ist.

## Claims

1. A system for reducing a distal fracture (2) of a long bone (3), between a distal fractured part (33) of the bone and a proximal part (36), comprising:
an osteosynthesis plate having a widened, pallet shaped epiphyseal part (4), provided with first fixing holes (6) for first anchoring screws (7), and an elongate diaphyseal part (8), provided with second fixing holes (9) for second anchoring screws (10), said plate having an articulation between the epiphyseal part and the diaphyseal part, in the plane of said diaphyseal part, between two defined angle values with respect to a central alignment position (18); and
a third anchoring screw (39),
the epiphyseal part (4) comprising on one side a first fixing portion forming a distal widened end thereof provided with said first fixing holes (6) on the distal part (33) of the fractured bone, and on the other side a second fixing portion forming a narrowed proximal end thereof,
**characterized in that** said narrowed proximal end is provided with a first slot (34) for fixing on the proximal part (36) of the bone adapted to receive the third anchoring screw (39), in order to fix the epiphyseal part (4) to each of the distal (33) and proximal (36) parts of the fractured bone (3) so that the epiphyseal part is rigidly fixed to the bone, wherein the first slot (34) is such that the distal and proximal parts of the fractured bone are movable relative to each other prior to insertion of the third anchoring screw into the distal part and immovable relative to each other after insertion of the third anchoring screw into the distal part,
and **in that** the narrowed end of the epiphyseal part further comprises a blocking screw (27) and a fixing screw (31), the blocking screw (27) defining an axis of rotation between the epiphyseal and diaphyseal parts and adapted for locking the epiphyseal part to the diaphyseal part, the fixing screw (31) being arranged for joining the epiphyseal part (4) to the diaphyseal part (8) through a second slot (30) provided in the diaphyseal part (8).

2. The system of claim 1, **characterized in that** it comprises means (24, 26, 27) for locking the epiphyseal part (4) to the diaphyseal part (8) in a chosen angular position.

3. The system of claim 2, **characterized in that** the means for locking the epiphyseal part (4) to the diaphyseal part (8) in the chosen angular position comprises surface blocking means (24, 26) spaced apart longitudinally with a system (11) for adjustable blocking by blocking screw (27).

4. The system of claim 3, **characterized in that**, in order to form the system (11) for adjustable blocking, the end of the epiphyseal part (4) for joining the epiphyseal part to the diaphyseal part (8) comprises a stud (29) opposite the second slot (30) situated in an end (21) of the diaphyseal part (8) for joining the diaphyseal part (8) to the epiphyseal part (4), with which it cooperates by sliding, and the fixing screw (31) fixes the stud on the end (21) of the diaphyseal part (8) through said second slot (30).

5. The system of claim 3, **characterized in that**, in order to form the surface blocking means, each of the epiphyseal and diaphyseal parts comprises an end (20, 21) for joining to the other part, each having a surface of cooperation (24, 26) with the other, and means (24, 26; 27, 31) for blocking said ends in rotation relative to each other in order to form said locking means.

6. The system of claim 5, **characterized in that** the mutually facing joining ends (20, 21) are made thin and superposed so as not to create an increased thickness with respect to the rest of the diaphyseal part once they have been joined.

7. The system of either of claims 5 and 6, **characterized in that** the blocking means comprises two opposite notched surfaces (24, 26), each being respectively integral with one end and able to turn relative to each other by a distance and to be blocked upon contact with each other, and a screw for engaging one of the notched surfaces to the other.

8. The system of any one of the preceding claims, **characterized in that** the diaphyseal part (8) is substantially elongate in a first plane, and the epiphyseal part (4) is situated mainly in a second plane inclined with respect to the plane of said diaphyseal part.

9. The system of any one of the preceding claims, **characterized in that** the two angle values defined in the diaphyseal plane are +15° and -15° with respect to the central alignment position.

10. The system of any one of the preceding claims, **characterized in that** the epiphyseal part (4) and the diaphyseal part (8) are movable relative to each other.

11. The system of any one of the preceding claims, **characterized in that** the diaphyseal part (8) comprises on one side an external end (21) adjacent the second fixing portion of the epiphyseal part, the external end (21) having the second slot (30) positioned in front of the narrowed proximal end (22) of the epiphyseal part, the second slot having a transverse dimension extending along the longitudinal axis, the narrowed proximal end of the epiphyseal part extending under the external end of the diaphyseal part and comprising a seat for a stud (29), at one end of the latter, said stud (29) having an axis located in said seat and a head extending above being in contact with the external end of the diaphyseal part and **in that** the second slot (30) is dimensioned to authorize an engagement of the stud with the epiphyseal part through the second slot during the relative rotation of the epiphyseal part and the diaphyseal part.

12. The system of claim 1, wherein the osteosynthesis plate further comprises a window (5) between the first fixing holes (6) and the first slot (34), **characterized in that** the window is configured for placement across the distal fracture (2) such that the distal fracture is viewable through the window when moving the distal part (33) and the proximal part (36) relative to each other.
